Europäisches Patentamt

European Patent Office

Office européen des brevets

(19)

(11) Numéro de publication : **0 146 462**
**B1**

(12) **FASCICULE DE BREVET EUROPÉEN**

(45) Date de publication du fascicule du brevet :
17.05.89

(21) Numéro de dépôt : 84402502.3

(22) Date de dépôt : 05.12.84

(51) Int. Cl.⁴ : **C 12 N 15/00**, C 12 P 21/02,
C 12 N 1/20 // (C12N1/20,
C12R1:19)

(54) Vecteurs de clonage et d'expression de l'interféron-y, bactéries transformées et procédé de préparation de l'interféron-y.

(30) Priorité : 09.12.83 FR 8319777

(43) Date de publication de la demande :
26.06.85 Bulletin 85/26

(45) Mention de la délivrance du brevet :
17.05.89 Bulletin 89/20

(84) Etats contractants désignés :
AT BE CH DE FR GB IT LI LU NL SE

(56) Documents cités :
EP–A– 0 041 767
EP–A– 0 089 676
EP–A– 0 094 887
EP–A– 0 095 350
EP–A– 0 099 084
EP–A– 0 110 044
GENE, vol. 13, no. 3, 1981, pages 289-298, Elsevier/North-Holland Biomedical Press; A. HONIGMAN et al.: "Plasmid vectors for positive selection of DNA inserts controlled by the lambda rho L promoter, repressor and antitermination function"

(73) Titulaire : TRANSGENE S.A.
16, rue Henri-Regnault
F-92400 Courbevoie (FR)

(72) Inventeur : Sondermeyer, Paul
24, rue des Acacias
F-67400 Ostwald (FR)
Inventeur : Courtney, Michael
c/o Transgene S.A. 95, rue Saint-Lazare
F-75009 Paris (FR)
Inventeur : Tessier, Luc-Henri
20, rue du Vieux Marché aux Grains
F-67000 Strasbourg (FR)
Inventeur : Lecocq, Jean Pierre
6, rue du Champ du Feu
F-67116 Reichsteet (FR)

(74) Mandataire : Warcoin, Jacques et al
Cabinet Régimbeau 26, avenue Kléber
F-75116 Paris (FR)

Jouve, 18, rue St-Denis, 75001 Paris, France

EP 0 146 462 B1

## Description

La présente invention concerne un procédé de préparation de l'interféron γ humain.

L'interféron γ (ci-après IFN-γ) est une glycoprotéine dotée in vitro d'une action anti-tumorale marquée. Il fait l'objet de nombreuses recherches au niveau pharmaceutique. Pour assurer le développement de ce produit, il convient de pouvoir le préparer en quantité importante afin d'en diminuer le prix.

En mettant en œuvre des techniques de génies génétiques on a pu décrire la préparation de l'IFN-γ par fermentation bactérienne notamment dans le demande de brevet Européen 0077670.

Toutefois il ne semble pas que les rendements obtenus soient particulièrement intéressants, ainsi dans le brevet mentionné précédemment l'activité du milieu fermenté est de $2,5 \times 10^5$U IFB-γ/1 ce qui est faible au niveau du rendement.

La présente invention a pour objet d'accroître le rendement de la fermentation par un facteur de 10 000 à 20 000, la quantité d'IFN-γ produit atteignant 20 % (du poids total) des protéines bactériennes produites.

Pour ce faire la présente invention propose des nouveaux vecteurs.

Il s'agit de vecteur d'expression de la protéine mature de l'interféron γ dans les bactéries du type comportant le gène codant pour la protéine mature de l'interféron γ humain et les éléments plasmidiques assurant l'expression de ce gène caractérisé en ce que l'extrémité 5' de la séquence codant pour la protéine mature est la suivante :

5' $\overline{\text{ATG}}$ TGC TAC TGT CAG GAT CCC 3'
TAC ACG ATG ACA GTC CTA GGG
Met Cys Tyr Cys Gln Asp Pro

Le début de la séquence originale et naturelle de IFN-γ est la suivante (moins le codon ATG de départ) :

ATG TGT TAC TGC CAG GAC CCA

en la comparant avec la séquence proposée :

$\overline{\text{ATG}}$ TGC$^*$ TAC TGT$^*$ CAG GAT$^*$ CCC$^*$

on note quatre changements de nucléotides qui conservent la composition de la protéine. Dans les exemples qui suivent on démontrera que ces changements permettent de multiplier par environ 10 000 le rendement en IFN-γ en particulier lorsque l'on utilise des vecteurs tels qu'ils seront décrits ci-après.

Les vecteurs selon la présente invention comportent outre le gène codant pour IFN avec une extrémité 5' comme indiquée précédemment :
— l'origine de réplication d'un plasmide bactérien ;
— un promoteur, en particulier tout ou partie d'un promoteur du bactériophage λ : $P_L$, $P_R$ ou $P'_R$ ;
— une région codant pour l'initiation de la traduction incorporant l'ATG de l'extrémité 5' du gène de IFN-γ.

La présence d'une origine de réplication pour un plasmide est essentielle pour permettre la réplication du vecteur dans les cellules bactériennes correspondantes, en particulier dans le cas de E. coli on utilisera, de préférence, l'origine de réplication du plasmide pBR322. Le plasmide pBR322 présente, en·effet, l'avantage de donner un grand nombre de copies et ainsi d'augmenter la quantité de plasmides produisant la protéine désirée.

Parmi les promoteurs du bactériophage λ, on utilisera, de préférence, le promoteur principal de gauche noté $\lambda P_L$. $P_L$ est un promoteur puissant responsable de la transcription précoce de λ.

Il est également possible d'utiliser d'autres promoteurs du bactériophage λ, notamment le promoteur de droite, $P_R$ ou le second promoteur de droite, $P'_R$.

Bien qu'il soit possible d'utiliser des séquences d'initiation de la traduction très variées, on préfère utiliser celle de la protéine cII du bactériophage λ qui sera nommée ci-après λcIIrbs.

Comme cela sera démontré il est également possible d'utiliser de telles séquences synthétiques en particulier tout ou partie de la séquence :

ATAACACAGGAACAGATCT$\overline{\text{ATG}}$

Le vecteur en cause comporte, en outre, de préférence une fonction d'antiterminaison de transcription codée par ex. par le gène N de λ noté λN. En présence du produit de transcription du gène N la transcription à partir de $P_L$ se poursuit au-delà de la plupart des signaux stop.

Ceci écarte les problèmes posés par un arrêt prématuré de la transcription qui peuvent se produire lorsque les gènes étrangers clonés présentent de tels signaux stop. En outre, il a été démontré que l'expression à partir de $P_L$ est améliorée dans un environnement $N^+$.

Afin d'écarter les problèmes de toxicité et d'instabilité du système hôte-vecteur en cas de production en continu de grandes quantités d'une protéine étrangère, il est nécessaire de prévoir le contrôle de l'activité du promoteur en lui adjoignant tout ou partie d'un système d'expression inductible, en particulier thermoinductible.

De préférence, le contrôle par la température de la synthèse de la protéine étrangère est effectué au niveau de la transcription au moyen d'un répresseur thermosensible codé dans la bactérie hôte, par exemple c1857, qui réprime l'activité de $P_L$ à 28 °C mais qui est inactivé à 42 °C. Le répresseur agit sur l'opérateur $O_L$ qui est adjacent au promoteur $P_L$. Bien que dans le cas précédent une partie du système d'expression thermoinductible soit partie intégrante de la bactérie hôte, il est possible de prévoir que ce système fasse partie du vecteur lui-même.

Le vecteur en cause peut également comporter un gène de résistance à un antibiotique, par exemple l'ampicilline dans le cas de pBR322, mais d'autres gènes de résistance peuvent être utilisés, résistance à la tétracycline (Tet$^r$) ou au chloramphénicol (Cm$^r$).

L'incorporation d'un tel marqueur est nécessaire pour la sélection des bactéries contenant les transformants porteurs du plasmide selon l'invention pendant les expériences de clonage.

L'incorporation d'un gène de résistance permet d'augmenter la stabilité du plasmide en imposant une pression de sélection lors de la fermentation, et en outre facilite l'isolement des transformants.

Pour le clonage il est intéressant de disposer d'un système permettant de détecter l'insertion dans un plasmide d'un ADN étranger.

A titre d'exemple, il est possible de prévoir dans la zone de clonage le fragment N-terminal de la β-galactosidase de E. coli (lac2') en le fusionnant avec la région d'initiation de traduction dérivée de λcII, ce qui met la traduction du fragment α sous le contrôle des séquences de cII.

Le fragment α est complémenté par l'expression du fragment ω C-terminal codé dans l'hôte, ceci conduit à une activité β-galactosidase dans les cellules. Cette activité β-galactosidase produit des colonies bleues en présence d'un substrat chromophorique, le 5-bromo-4-chloro-3-indolyl-β-D-galactosidase.

A 28 °C, le promoteur $P_L$ est inactivé, le fragment α n'est pas synthétisé et les colonies demeurent blanches. Lorsque la température est amenée à 42 °C, le promoteur $P_L$ est activé, le fragment α est synthétisé et les colonies virent au bleu.

L'insertion d'ADN étrangers dans les sites de clonage situés dans ce système de détection empêche la synthèse de la β-galactosidase et conduit donc à des colonies blanches aussi bien à 28 °C qu'à 42 °C.

Il est également possible de remplacer le gène lacZ' par d'autres gènes permettant une détection.

La présente invention concerne en outre les bactéries notamment les souches de E. coli transformées par les vecteurs selon l'invention par des techniques connues et dont certaines seront rappelées dans les exemples.

Enfin l'invention concerne un procédé de préparation de IFN-γ humain dans lequel on cultive sur un milieu de culture des bactéries transformées comme décrit précédemment et dans lequel on récupère ensuite l'IFN-γ formé.

Les milieux de culture mis en œuvre sont connus de l'homme de métier et devront être adaptés à chaque souche cultivée. La culture sera de préférence effectuée en présence de l'antibiotique à l'encontre duquel la souche transformée est devenue résistante.

IFN-γ est séparé après éclatement des cellules par des techniques connues comme la séparation sur colonne d'affinité ou la chromatographie d'exclusion.

La présente invention comporte, bien entendu, d'autres aspects, notamment certains plasmides qui seront décrits dans les exemples ainsi que leurs mutants et dérivés et de façon générale les procédés de fermentation des bactéries transformées ainsi que l'IFN-γ ainsi obtenu.

D'autres caractéristiques et avantages de l'invention seront mieux compris à la lecture des exemples ci-après et des dessins ci-annexés sur lesquels :

la figure 1   représente la stratégie permettant de préparer le plasmide pTG907,
la figure 2   représente la stratégie permettant de préparer le phage M13tg910,
la figure 3   représente la structure du phage M13tg910,
la figure 4   représente la stratégie permettant de préparer le plasmide pTG908,
la figure 5   représente la séquence complète du gène codant pour IFN-γ isolé à partir de la banque,
la figure 6   représente la stratégie de préparation de pTG909,
la figure 7   représente la stratégie de préparation de pTG941,
la figure 8   représente la stratégie de préparation de pTG951.

Il convient de remarquer que les différentes séquences de nucléotides figurant dans les dessins doivent être considérées comme faisant explicitement partie de la présente description, ces séquences n'ont pas été reproduites dans le corps du texte afin de ne pas l'alourdir inutilement.

1) Procédés généraux

a) Souches bactériennes

Les souches bactériennes utilisées dans le cadre de la présente invention sont les suivantes :
TGE900 qui est une souche de E. coli ayant les caractéristiques suivantes : su⁻F⁻his ilv bio (λcI857ΔBamΔHI).

N6437, souche de E. coli ayant les caractéristiques suivantes : F⁻his ilv gal⁺ Δ8proC⁺ : tn10 lacΔm15 (λcl857ΔBamΔHI).

Jm103 qui est une souche de E. coli ayant les caractéristiques suivantes : Δ(lac-pro) sup$^E$ thi endA sbcB15 sttA rK⁻ nK⁺/F¹ traD36 proAB⁺ laci$^a$ lacZΔm15.

Les souches mentionnées précédemment ont été utilisées parce qu'elles étaient disponibles, mais il est bien entendu qu'il est possible d'utiliser d'autres souches dans la mesure où elles présentent certaines caractéristiques essentielles qui sont rappelées dans le cours de la description détaillée.

b) Préparation des ADN

Des mini-préparations d'ADN de plasmides ou de phages M13 sont effectuées comme cela est décrit par Ish-Horowitz (référence 1) à la seule différence que l'ADN est précipité une seconde fois avec de l'éthanol avant d'être utilisé.

Les maxi-préparations sont effectuées comme cela est décrit dans la publication précédente, avec une purification complémentaire par un gradient de densité CsCl/bromure d'éthidium.

c) Techniques de clonages

Le traitement des ADN avec des enzymes de restriction est effectué, sauf indications contraires, en utilisant les conditions indiquées par le fabricant (New England Biolabs, Bethesda Research Laboratories et Boehringer Mannheim).

Lorsque cela est nécessaire, les phosphates des extrémités 5′ sont éliminés en utilisant, soit une phosphatase alcaline bactérienne, soit une phosphatase d'intestin de veau, pendant 30 minutes à 37 °C dans le tampon d'enzyme de restriction.

La réparation des extrémités cohésives utilisant la polymérase de Klenow (Boehringer Mannheim) est effectuée à 25 °C pendant 15 minutes dans un mélange de 50 mMol. Tris HCl, pH 7,8, 5 mMol. MgCl₂, 10 mMol. de β-mercaptoéthanol, 0,4 mMol. de dNTPs avec l'enzyme et 10 à 200 µg/ml d'ADN.

La nucléase S₁ (Miles) est utilisée à 2 u/µg d'ADN à 25 °C pendant 30 minutes dans un milieu 0,3 Mol. NaCl, 0,03 Mol. NaOAc, pH 4,8, 0,003 Mol. ZnCl₂.

Bal31 est utilisée selon le procédé de Panayotatos et al. (référence 2). Les ligations sont effectuées à 15 °C (sauf lorsque cela est indiqué différemment) pendant 4 à 24 heures en utilisant de l'ADN ligase T₄ (Boehringer Mannheim) avec 100 mMol. de NaCl, 66 mMol. de Tris HCl, pH 7,5, 10 mMol. de MgCl₂, 0,5 mMol. de spermidine, 0,2 mMol. de EDTA, 2 mMol. de DTT, 1 mMol. d'ATP, 0,1 mg/ml de BSA et 5 à 50 µg/ml d'ADN.

Pour la ligation d'extrémités cohésives, on utilise environ 30 unités/ml de ligase. Pour la ligation des extrémités franches on utilise environ 100 unités/ml de ligase.

Entre les différentes réactions enzymatiques, des échantillons d'ADN sont extraits avec un mélange phénol/chloroforme puis précipités à l'éthanol. Lorsque cela est nécessaire, du tARN de E. coli ou de levures est utilisé comme entraîneur. Les adaptateurs moléculaires (Collaborative Research, Bethesda Research Laboratories, New England Biolabs) sont préhybridés et utilisés en excès molaire de 10 à 50 fois pour les extrémités franches d'ADN utilisant les conditions de tampon décrites précédemment avec 100 unités/ml de ligase T₄ à 4 °C pendant 15 heures. Lorsque l'on utilise des adaptateurs non phosphorylés, les adaptateurs qui n'ont pas réagi sont éliminés directement après ligation par précipitation avec le tétrachlorhydrate de spermine (Hoopes et al., référence 3).

Lorsque l'on utilise des adaptateurs phosphorylés, le mélange de ligation est tout d'abord extrait avec un mélange phénol/chloroforme puis précipité avec de l'éthanol avant coupure spécifique avec les enzymes de restriction appropriées puis précipitation avec le tétrachlorhydrate de spermine.

Les cellules bactériennes compétentes sont préparées puis transformées avec les plasmides ou transfectées par l'ADN de M13 selon les procédés décrits par Dagert et Ehrlich (référence 4).

Exemple 1

La préparation du pTG908 implique tout d'abord la préparation d'un plasmide comportant :

— l'origine de réplication de pBR322,
— le gène de résistance à l'ampicilline de ce même plasmide(amp$^R$),
— le promoteur P$_L$ et le gène λN.

1) Suppression du site Pstl dans pBR322

Le plasmide de base utilisé est le plasmide pBR322 ; toutefois, celui-ci présente l'inconvénient d'avoir à l'intérieur du gène amp$^R$ un site de restriction Pstl, car un site de même nature sera utilisé par la suite dans la zone de clonage comme site unique de restriction. Il convient donc de faire disparaître ce site de restriction Pstl en utilisant un mutant du plasmide pBR322, le plasmide pUC8, dans lequel le gène de résistance à l'ampicilline ne présente pas de site de restriction Pstl (ce site a été éliminé par mutation in vitro). pBR322 est commercialisé notamment par Bethesda Research Laboratories et pUC8 est décrit dans l'article référencé 5.

Pour ce faire, on échange le fragment PvuI/PvuII de 1 669 bp de pBR322 avec le fragment analogue PvuI/PvuII du plasmide pUC8. Afin de réaliser cet échange les plasmides pBR322 et pUC8 sont traités successivement par PvuI, PvuII, puis circularisés par section d'une ligase.

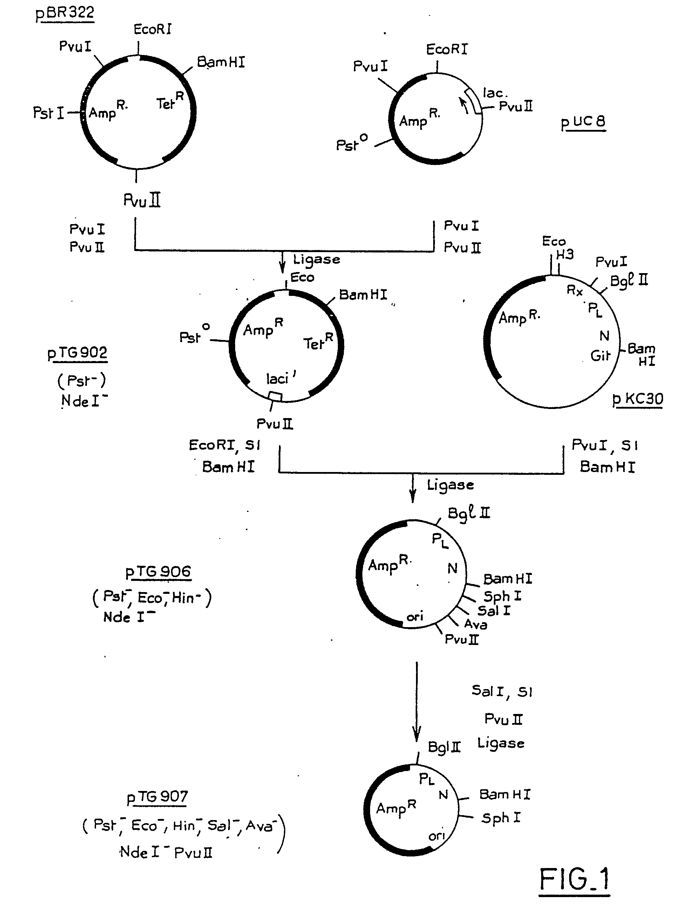

On obtient ainsi le plasmide pTG902 qui ne présente plus de site de restriction PstI et qui a également perdu le site de restriction NdeI présent à l'origine sur pBR322 (non représenté sur la fig. 1). En outre, le plasmide pTG902 porte un fragment de 50 bp correspondant à la séquence lacI' dans lequel se trouve le site PvuII.

2) Insertion du promoteur $P_L$ et du gène $\lambda N$ et préparation du plasmide de pTG907

Le promoteur $P_L$ et le gène $\lambda N$ sont isolés du plasmide pKC30 pour être insérés dans pTG902, le segment prélevé contient également l'opérateur $O_L$ sur lequel agira le répresseur thermosensible cI850, comme cela sera décrit dans les essais. En outre, le procédé permet de supprimer les sites EcoRI et HindIII tout en conservant un site unique BamHI en aval du gène N pour pouvoir ensuite insérer $\lambda$cIIrbs. Le plasmide de pKC30 est décrit dans la référence 6.

pTG902 est coupé en son site de restriction unique EcoRI et les extrémités 5' sortantes sont éliminées par traitement à la nucléase $S_1$. Après une digestion avec BamHI, le fragment le plus important est purifié sur gel.

Le fragment portant le promoteur $P_L$ et le gène $\lambda N$ est préparé de la même façon à partir de pKC30 en traitant successivement le plasmide par PvuI, la nucléase $S_1$ et BamHI. Les fragments, après purification sur gel, sont soumis à l'action de la ligase, ce qui conduit à la fusion EcoRI/PvuI et à la reconstitution du site BamHI.

Le mélange de ligation est utilisé pour transformer des cellules hôtes compétentes, TGE900, à 30 °C. Cette souche contient le prophage $\lambda$ délété, $\lambda$cI857$\Delta$Bam$\Delta$HI, qui fournit le répresseur de $\lambda$ thermosensible, cI857, qui est nécessaire pour bloquer la transcription à partir de $P_L$.

Ceci est important car l'activité de $P_L$ dans un milieu $N^+$ est léthale compte tenu de la fonction d'antiterminaison de N.

Après analyse par enzymes de restriction, les clones contiennent des plasmides de structure correcte et sont ensuite testés pour leur manque de viabilité à 42 °C.

L'un des plasmides obtenus, pTG906, est traité de façon à éliminer le segment PvuII-SalI de manière à supprimer les sites de restriction compris sur ce segment et afin de faire disparaître également les deux sites de restriction extrêmes. Pour ce faire, pTG906 est traité successivement avec SalI, la nucléase $S_1$, PvuII et la ligase.

On obtient ainsi le plasmide pTG907 qui comporte l'ensemble des éléments évoqués au début de cette étape et, en outre, qui est Pst⁻ Eco⁻ Hind⁻ Sal⁻ Ava⁻ Nde⁻ PvuII⁻. La synthèse de ce plasmide est représentée sur la figure 1.

3) Clonage de la région $\lambda$cIIrbs

La seconde phase importante de la synthèse consiste à insérer la région $\lambda$cIIrbs sous forme d'un fragment AvaI/TaqI dans le début du gène lacZ' (fragment $\alpha$ de la $\beta$-galactosidase) lequel a été cloné dans le phage M13 nommé M13tg110. Cette stratégie permet un test fonctionnel simple pour rbs, à savoir la production de la protéine lacZ' et, par conséquent, d'obtenir des plaques bleues en présence d'IPTG et de Xgal ; ceci permet également un séquençage rapide de la construction en utilisant la méthode dite du didéoxy.

Dans le cours des expériences, différents dérivés du phage M13 seront mentionnés. Précédemment on a mentionné le phage M13tg110 et dans la suite de la description on utilisera d'autres phages du même type dont la construction va être rappelée ci-après.

La construction de ces vecteurs est indiquée dans le tableau I ci-après sur lequel figurent les références du phage de départ, la nature de l'enzyme de restriction avec laquelle il a été découpé, le traitement particulier qu'ont subi les fragments ainsi obtenus et la nature de l'insert qui a été fixé dans les sites ainsi révélés.

Le phage M13mp7 est commercialisé notamment par la société Bethesda Research Laboratories.

M13mp701 est obtenu à partir de M13mp7 par remplacement du fragment PstI/EcoRI à droite (CTG CAG... GAA TTC) par la séquence : CTG CAG CAA TTC.

Le principe de la construction est décrit dans le schéma suivant :

(Voir schéma et tableau I pages 6 et 7)

ATG ACC ATG ATT ACG AAT TCC CCG GAT CCG TCG ACC TGC AGC AAT TCA CTG GCC    M13mp701

BamHI ↓

ATGACCATGATTACGAATTCCCCG        GATCCGTCGACCTGCAGCAATTCACTGGCC

TACTGGTACTAATGCTTAAGGGGCCTAG        GCAGCTGGACGTCGTTAAGTGACCGG

DNA polymérase ↓

ATGACCATGATTACGAATTCCCCGGATC ↓ GATCCGTCGACCTGCAGCAATTCACTGGCC

TACTGGTACTAATGCTTAAGGGGCCTAG        CTAGGCAGCTGGACGTCGTTAAGTGACCGG

Linker HindIII ↓

ATGACCATGATTACGAATTCCCCGGATCCCAAGCTTGG   CCAAGCTTGGGATCCGTCGACCTGCAGCAATTCACTGGCC

TACTGGTACTAATGCTTAAGGGGCCTAGGGTTCGAACC   GGTTCGAACCCTAGGCAGCTGGACGTCGTTAAGTGACCGG

HindIII ↓

ATGACCATGATTACGAATTCCCCGGATCCCA        AGCTTGGGATCCGTCGACCTGCAGCAATTCACTGGCC

TACTGGTACTAATGCTTAAGGGGCCTAGGGTTCGA        ACCCTAGGCAGCTGGACGTCGGTTAAGTGACCGG

T$_4$DNA ligase ↓

ATG ACC ATG ATT ACG AAT TCC CCG GAT CCC AAG CTT GGG ATC CGT CGA CCT GCA GCA ATT CAC TGG CC

| EcoRI | BamHI | HindIII | BamHI | SalI | PstI |
|-------|-------|---------|-------|------|------|

EP 0 146 462 B1

Tableau I

| Nom | Phage parental | Site de clivage | Traitement | Insert | Phase de HindIII | Phase de BamHI | Phase de PstI | Complémentation de lacα +/- |
|---|---|---|---|---|---|---|---|---|
| M13mp701 | M13mp7 | Construction | D.R. Bentley | | O | I | I | + |
| M13tg102 | M13mp701 | AccI | ADN polymérase | séquence HindIII(a) | I | I | I | + |
| M13tg110 | M13mp701 | AccI | $S_1$ nucléase | séquence HindIII(a) | III | I | II | − |
| M13tg115 | M13tg110 | EcoRI | $S_1$ nucléase | séquence BglII (b) | II | III | I | + |

(a) Adaptateur de séquence HindIII phosphorylé CCAAGCTTGG
(b) Adaptateur de séquence BglII non phosphorylé CAGATCTG

EP 0 146 462 B1

Les protocoles d'utilisation des enzymes (restriction, ADN polymérase, T$_4$ ADN ligase) sont ceux indiqués dans les notices des fournisseurs. Le « linker » HindIII est un oligonucléotide synthétique de séquence 5'/p CCAAGCTTGG 3' (Collaborative Research Inc., Waltham, Mass 02154, E.U.A.).

La région cIIrbs est isolée à partir d'un plasmide pOG11 (réf. 7) qui contient un fragment de λHaeIII/Sau3A qui s'étend du milieu du gène cro jusqu'au milieu de la région codant pour CII (cro et CII étant impliqués notamment dans la régulation de la lysogénie du bactériophage λ).

On prélève dans pOG11 le fragment AvaI/TagI de 186 bp contenant la région cIIrbs et on le traite avec la polymérase Klenow. D'autre part, on traite le phage M13tg110 par BamHI puis par la polymérase de Klenow suivi d'un traitement à la phosphatase intestinale de veau. Les fragments obtenus sont soumis à l'action de la ligase T$_4$.

Un examen des séquences du fragment de pOG11 comportant la région cIIrbs et du phage M13tg110 permet de prévoir que l'insertion du fragment portant cIIrbs dans le site BamHI de M13tg110 doit conduire à l'obtention de plaques bleues après fermentation des bactéries transfectées compte tenu du fait que le gène lacZ' est en phase pour la traduction à partir de AUG du gène de cII, tandis que les plaques correspondant à la souche parentale M13tg110 sont blanches.

Les plaques bleues sont prélevées puis les colonies sont sélectionnées par analyse de restriction enzymatique des mini-préparations et l'on vérifie ensuite par séquençage que la construction obtenue est correcte.

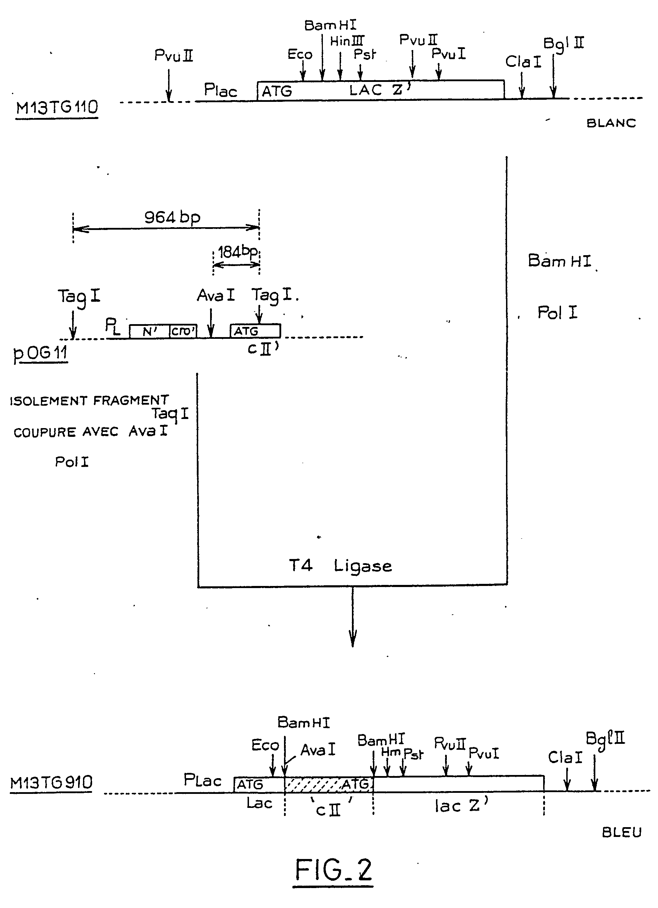

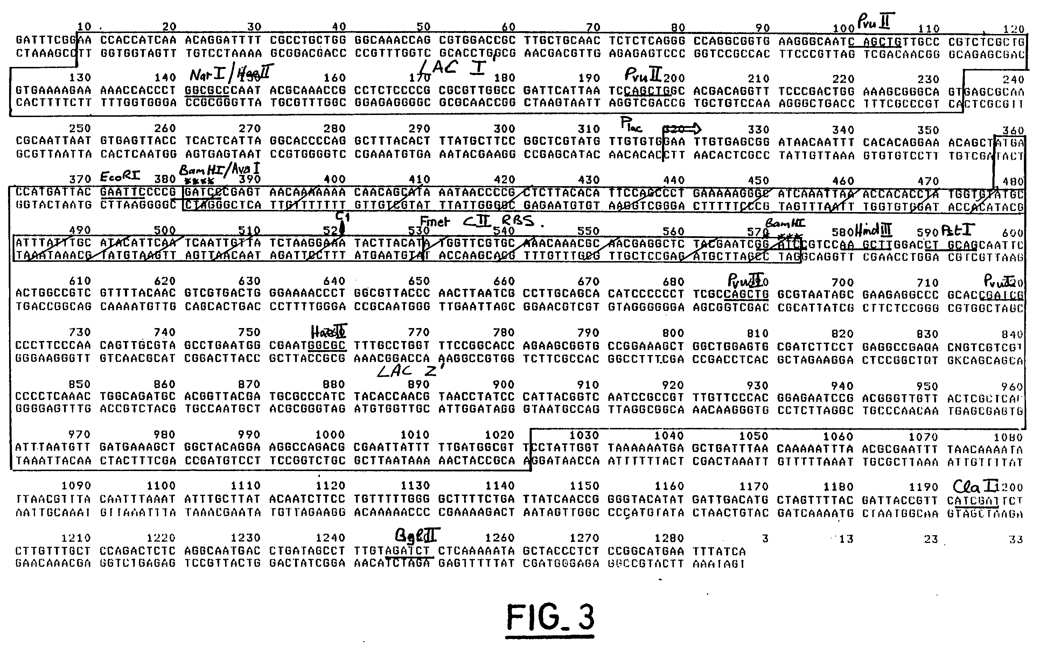

On obtient ainsi un clone résultant M13tg910 dont la structure globale est représentée à la partie inférieure de la Figure 2 et la structure détaillée est représentée à la figure 3.

On constate que l'insertion du fragment cIIrbs reconstruit en amont les sites BamHI et AvaI et en aval le site BamHI.

La traduction à partir de AUG de cII conduit à la fusion des 13 aminoacides terminaux de cII aux 8 aminoacides du NH$_2$ terminal de la protéine lacZ'.

4) Insertion du fragment λcIIrbs dans le plasmide pTG907

La troisième étape de cette synthèse consiste à transférer le fragment cIIrbs/lacZ' du phage M13tg910 sur le plasmide vecteur pTG907 préparé précédemment.

Pour ce faire, il convient tout d'abord d'éliminer les sites EcoRI, BamHI et AvaI en amont de cIIrbs puis d'insérer un site Bg1II.

Dans ces conditions, cIIrbs peut être prélevé sous forme d'un fragment Bg1II-Bg1II et placé dans le site BamHI en aval du promoteur P$_L$ et du gène λN de pTG907.

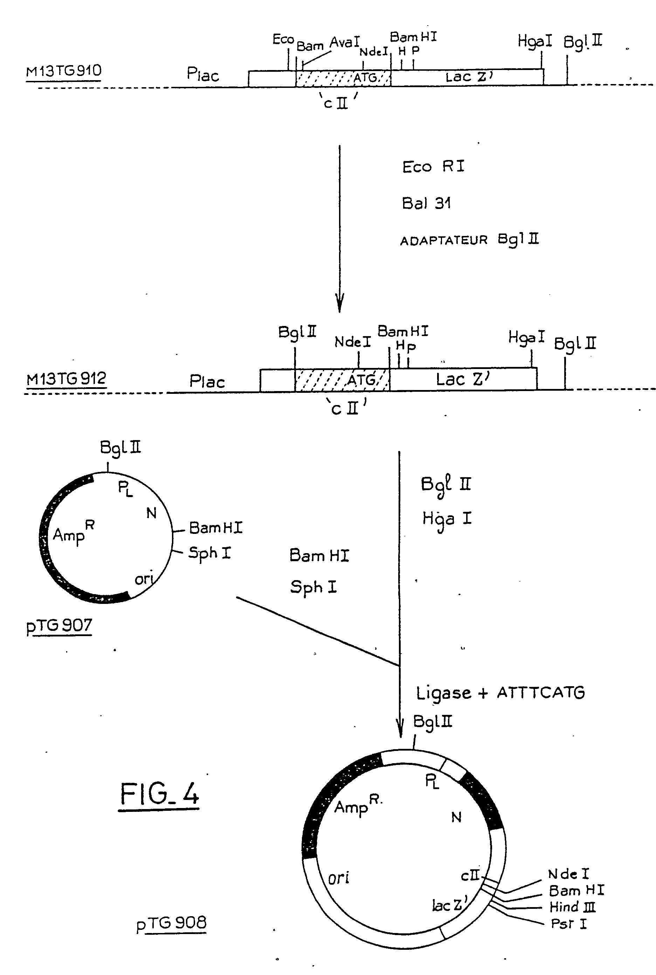

Le phage M13tg910 est digéré avec EcoRI puis traité avec Bal31 puis ensuite par la polymérase de Klenow. Les fragments obtenus sont alors soumis à l'action de la ligase en présence d'adaptateurs Bg1II non phosphorylés. Le mélange de ligation obtenu est utilisé pour transformer des cellules compétentes JM103. On sélectionne alors les plages bleues. Ces clones sont ensuite analysés afin de vérifier qu'ils contiennent le site Bg1II et qu'ils ne présentent plus de site EcoRI ou BamHI en amont. On obtient ainsi des clones tels que M13tg912 dont la structure est représentée sur la figure 4.

Le traitement par Ba131 a produit une délétion de 101 bp éliminant les sites EcoRI, BamHI et AvaI ; ainsi que les séquences de lac ATG et lac Shine/Dalgarno. Le site Bg1II introduit se trouve placé enrion 100 bp en amont de l'ATG de cII et 10 bp en aval de P$_{lac}$.

Le fragment BamHI/SphI de pTG907, le fragment Bg1II/Hgal portant cIIrbs et lacZ' et l'adaptateur phosphorylé ont été préhybridés dans un rapport molaire de 1 : 2 : 1 puis traités avec la ligase T$_4$. Des aliquots sont utilisés pour transformer les cellules compétentes de la souche 6150 à 30 °C.

Les cellules intéressantes sont identifiées en sélectionnant les transformants avec un fragment cIIrbs/lacZ', marqué au P$^{32}$ et la construction obtenue est confirmée par une étude de restriction enzymatique.

Afin d'avoir une première indication montrant que les différents éléments du système d'expression se conduisent comme cela est désiré, le plasmide obtenu, pTG908, est transféré dans une souche hôte N6437 qui possède à la fois c1857 et le fragment ω de la β-galactosidase complémentant le fragment α qui est codé par le plasmide.

Les transformants obtenus placés sur une boîte contenant IPTG + Xgal sont blancs à 28 °C puis virent au bleu environ 30 minutes après lorsqu'on les transfère à 42 °C.

Exemple 2 : Sélection du clone de cDNA de IFN-γ humain

On réalise selon les procédés connus une banque de cDNA à partir des mARN de lymphocytes induits par des agents mitogènes.

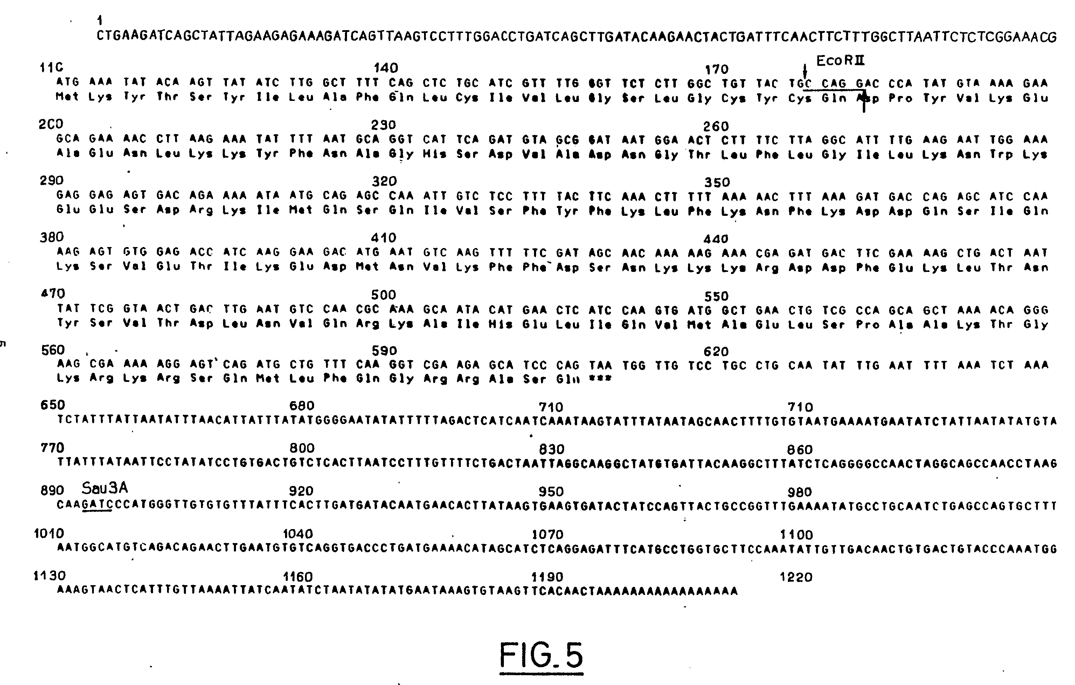

Puis on sélectionne un clone comportant la séquence codant pour IFN-γ complet (figure 5) ce clone est dénommé pTG11.

IFN-γ est synthétisé sous forme d'un prépeptide dont la séquence signal de 20 amino-acides est scindée pour donner le polypeptide mature qui débute par la séquence cys-tyr-cys et couvre les amino-acides 21 à 166.

L'analyse de la séquence des nucléotides pour les sites de restriction révèle un site EcoRII à 8 bp en aval du départ de la protéine mature et un site Sau3A à 285 bp en aval du codon stop, ce qui permet d'isoler pratiquement toute la séquence codant pour la protéine mature sur un fragment EcoRII/Sau3A.

### Exemple 3 : Construction de pTG909 plasmide expirant faiblement IFN-γ humain

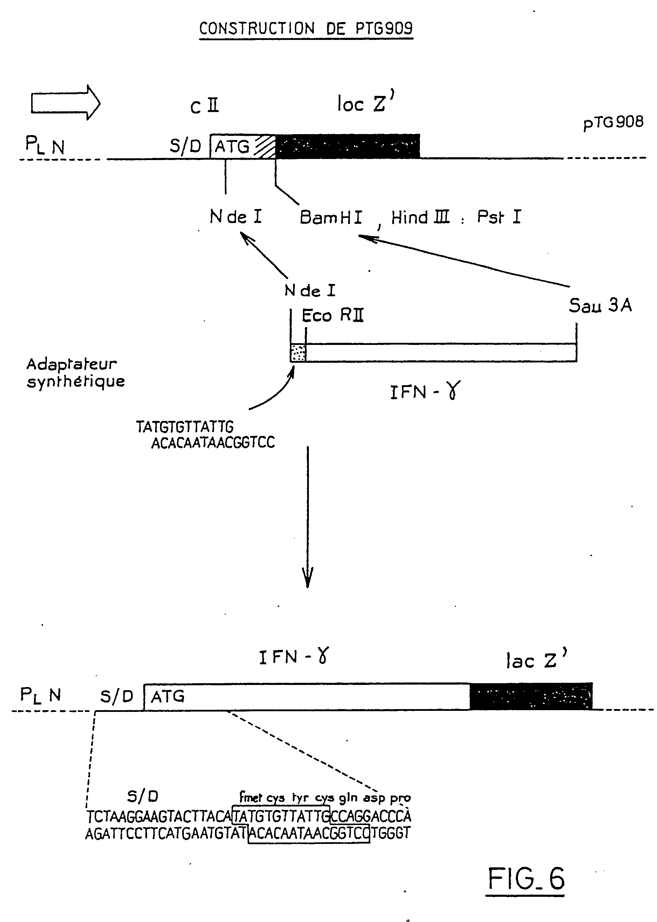

La figure 6 schématise la préparation de pTG909.
On utilise tout d'abord une molécule d'adaptation synthétique qui permet :
a) d'effectuer la jonction entre les extrémités EcoRII et Ndel,
b) d'introduire les 8 bp manquantes par rapport à la séquence codant pour IFN-γ mature et,
c) de reconstituer le codon de départ ATG de cIIrbs de façon que la séquence codant pour la protéine IFN-γ mature soit traduite sans amino-acides fusionnés à l'exception de l'initiateur F-met.
Cet adaptateur est synthétisé chimiquement et sa constitution est représentée sur la figure 2.
pTG11 est mis en digestion avec EcoRII et Sau3A et pTG908 avec Ndel et BamHI.
Les fragments appropriés sont purifiés sur gel, mélangés avec une quantité équimolaire de l'adaptateur, préhybridés et ligés. Le mélange est utilisé pour transformer des cellules compétentes TGE900 et les transformants sont sélectionnés en hybridant un insert PstI de pIG11 « nick-traduit » et marqué au $p^{32}$ avec les transformants.
13 clones sont sélectionnés et contrôlés par cartographie et l'un d'eux pTG909 est vérifié par séquençage.
La mise en culture du clone de TG900 transformé par TG909 est effectuée sur un milieu LB avec 50 µg/ml Ampicilline jusqu'à une densité optique $DO_{660} = 0,3$ ($\sim 10^8$ cellules/ml) et induite pendant 1 heure à 42 °C. Les extraits sont préparés pour tester leur activité IFN-γ selon des procédés connus :
Les résultats montrent une activité de $10^5$ unité/l de culture en IFN-γ. Le poids moléculaire de la protéine d'environ 17 000 dalton est en bon accord avec ce que l'on sait de IFN-γ.
Mais cette activité correspond à environ 0,001 % du contenu total en protéine de la cellule.
C'est ce résultat qui a amené à réétudier la structure fine des bases au voisinage de codon de départ et à proposer des mutations ponctuelles conservatrices permettant d'améliorer le rendement en IFN-γ.

### Exemple 4 : Construction du vecteur pTG941

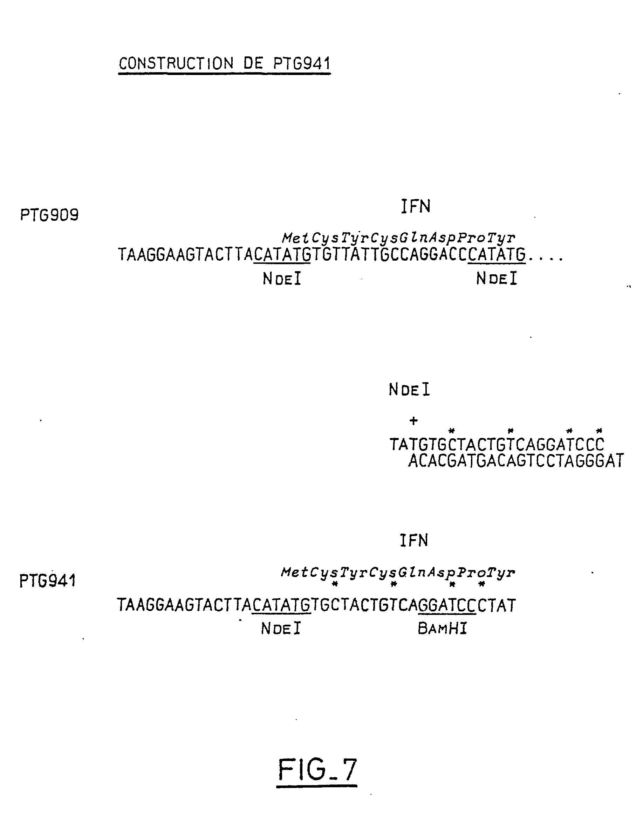

pTG909 contient 2 sites Nde1, l'un au codon de départ de IFN-γ et l'autre 22 bp plus tard dans la séquence IFN-γ (voir figure 7).
La région entre ces sites qui est la région codant pour les 7 premiers amino-acides de IFN-γ a été éliminée par traitement avec Ndel et remplacée par un oligonucléotide synthétique qui est représenté sur la figure 7.
Cette réaction détruit le site Ndel aval et reconstitue le site Ndel amont tout en introduisant un site BamHI qui est unique.
Ce changement de base est conservateur, c'est-à-dire que la séquence d'amino-acide n'est pas altérée.
Les extraits obtenus dans les mêmes conditions que précédemment à partir de souche transformée par le plasmide pTG941, contiennent $2 \times 10^9$ U/l IFN-γ.
Ce résultat est 10 000 fois supérieur aux résultats obtenus avec pTG909 et correspond à 10 % du poids total des protéines cellulaires produites.

### Exemple 5 : Construction de pTG951

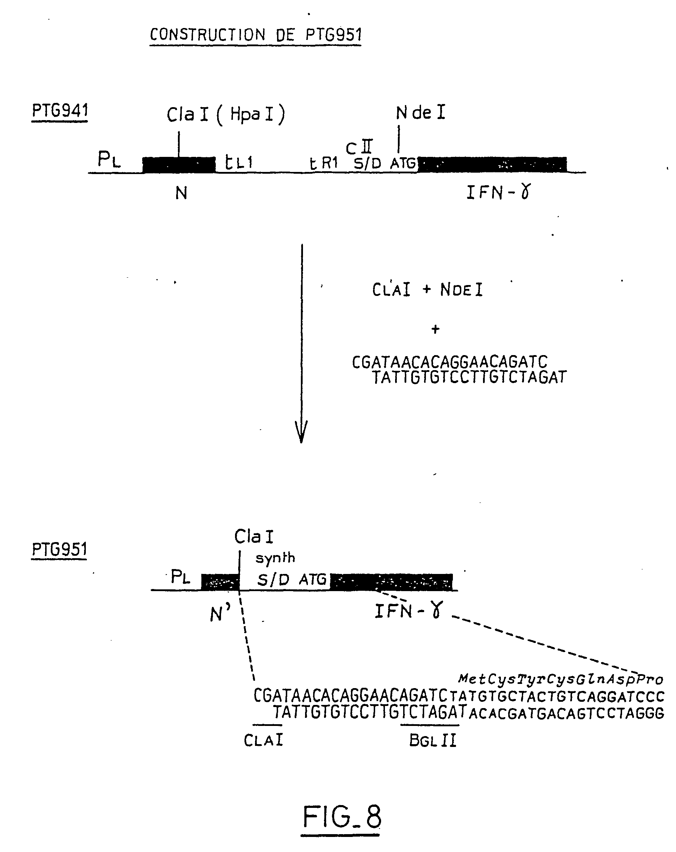

La figure 8 schématise la construction de pTG951 qui est un dérivé de pTG941 dans lequel un fragment contenant le cIIrbs a été remplacé par une séquence synthétique sur la base de la séquence de la région d'initiation de la traduction de l'opéron E. coli lac noté E. coli lac opéron rbs. Cet oligonucléotide synthétique a été inséré entre le site unique Ndel du codon de départ de la séquence codant pour IFN-γ et le site ClaI qui a été inséré au niveau du site HgaI dans le gène N. De ce fait par traitement avec Ndel et ClaI le plasmide pTG951 ne contient plus qu'un gène N tronqué (un codon stop en phase avec la traduction du gène N est placé immédiatement en amont du nouveau site rbs) et est dépourvu des terminateurs de transcription tL1 et tR1 présents dans pTG909 et pTG941.
Les extraits bactériens obtenus à l'aide de bactéries transformées par pTG951 conduisent à une production de $5 \times 10^9$ IFN-γ U/l de culture, ce qui correspond à 25 % du poids total des protéines cellulaires produites.
Le site rbs synthétique de pTG951 contient un site BglII unique immédiatement avant le codon de départ, il est donc possible de faire des dérivés de pTG951 par scission avec BglII suivi par diverses manipulations utilisant soit l'ADN polymérase I ou la nucléase S1. Ces dérivés présentent des variations dans la distance et les séquences entre la séquence de Shine/Dalgarno et l'ATG. Mais aucun des plasmides ainsi préparé ne présente une activité supérieure au pTG951 lui-même.
Les principaux résultats sont rappelés dans le tableau ci-après :

Tableau

| NOM | PROMOTEUR | RBS | SEQUENCE DE RBS ET JONCTION AVEC LA SEQUENCE IFN | UIFNγ/l | % PROTEINE |
|---|---|---|---|---|---|
| pTG909 | PL | cII | fmet cys tyr cys gln asp pro tyr<br>TAAGGAAGTACTTACATATG TGT TAT TGC CAG GAC CCA TAT | $10^5$U | NON DETECTE |
| pTG941 | PL | cII | fmet cys tyr cys gln asp pro tyr<br>TAAGGAAGTACTTACATATG TGC TAC TGT CAG GAT CCC TAT | $2 \times 10^9$U | >10 % |
| pTG951 | PL | SYNTH (lac) | fmet cys tyr cys gln asp pro tyr<br>CACAGGAACAGAGATCTATG TGC TAC TGT CAG GAT CCC TAT<br>------<br>BglII | $5 \times 10^9$U | >10 % |

EP 0 146 462 B1

Bibliographie

1. Ish-Horowitz D. et Burke J. F., Nucl. Acids Res. 9, 2989-2998 (1981).
2. Panayotatos N. et Trong K., Nucl. Acids Res. 9, 5679-5688 (1981).
3. Hoopes B. C. et McClure R. R., Nucl. Acids Res. 9, 5493-5504 (1981).
4. Dagert M. et Ehrlich S. D., Gene, 23-28 (1979).
5. Vieira J. et Messing J., Gene 19, 259-268.
6. Shimatake H. et Rosenberg M., Nature, 292, 128-132, (1981).
7. Oppenheimer A. B., Gottesman S. et Gottesman M., J. Mol. Biol., 158, 327-346 (1982).

**Revendications**

1. Vecteur d'expression de la protéine mature de l'interféron γ dans les bactéries du type comportant la séquence nucléotidique codant pour la protéine mature de l'interféron γ humain et les éléments plasmidiques assurant l'expression de cette séquence nucléotidique caractérisé en ce que l'extrémité 5' de la séquence codant pour la protéine mature est la suivante :

5' ATG TGC TAC TGT CAG GAT CCC 3'
   TAC ACG ATG ACA GTC CTA GGG
   Met Cys Tyr Cys Gln Asp Pro

2. Vecteur de clonage selon la revendication 1 caractérisé en ce qu'il comporte en outre :
a) l'origine de réplication d'un plasmide bactérien,
b) un promoteur,
c) une région d'initiation de la traduction comportant le codon ATG de la séquence codant pour IFN-γ mature.

3. Vecteur selon la revendication 2 caractérisé en ce qu'il comporte :
a) l'origine de réplication d'un plasmide bactérien,
b) un promoteur du bactériophage λ, $P_L$, $P_R$ ou $P'_R$,
c) une région d'initiation de la traduction choisie parmi tout ou partie λcIIrbs ou une séquence synthétique placée sous le contrôle du promoteur du bactériophage λ.

4. Vecteur selon la revendication 2 caractérisé en ce que la région d'initiation de la traduction est λcIIrbs.

5. Vecteur selon la revendication 2 caractérisé en ce que la région est constituée par tout ou partie de la séquence :

ATAACACAGGAACAGATCTATG.

6. Vecteur selon l'une des revendications 1 à 5 caractérisé en ce que le promoteur utilisé est le promoteur $P_L$.

7. Vecteur selon l'une des revendications 1 à 6 caractérisé en ce qu'il comporte, en outre, une fonction d'antiterminaison de transcription.

8. Vecteur selon la revendication 7 caractérisé en ce que la fonction d'antiterminaison de transcription est le gène λN.

9. Vecteur selon l'une des revendications 1 à 8 caractérisé en ce qu'il comporte l'origine de réplication de pBR322.

10. Vecteur selon l'une des revendications 1 à 9 caractérisé en ce qu'il comporte un gène codant pour la résistance à un antibiotique.

11. Vecteur selon la revendication 10 caractérisé en ce qu'il comporte un gène de résistance à l'ampicilline.

12. Bactéries transformées par un vecteur selon l'une des revendications 1 à 11.

13. Bactéries selon la revendication 12 caractérisé en ce qu'il s'agit de E. coli.

14. Procédé de préparation d'interféron γ humain caractérisé en ce qu'on a cultivé sur un milieu de culture une bactérie selon l'une des revendications 12 et 13 et en ce que l'on isole IFN-γ humain obtenu après culture.

**Claims**

1. Vector for the expression of the mature protein of γ interferon in bacteria, of the type containing the nucleotidic sequence which codes for the mature protein of γ interferon and the plasmid elements which provide for the expression of this nucleotidic sequence wherein the 5' end of the sequence coding for the mature protein is as follows :

5´ATG TGC TAC TGT CAG GAT CCC 3´
TAC ACG ATG ACA GTC CTA GGG
Met Cys Tyr Cys Gln Asp Pro

2. Cloning vector as claimed in claim 1, which contains in addition,
   a) the origin of replication of a bacterial plasmid,
   b) a promoter,
   c) a translation initiation region containing the ATG codon of the sequence coding for mature γ-IFN.
3. Vector as claimed in claim 2, which contains :
   a) the origin of replication of a bacterial plasmid,
   b) a bacteriophage λ promoter, P_L, P_R or P´_R,
   c) a translation initiation region chosen from all or part of λcIIrbs or a synthetic sequence placed under the control of the bacteriophage λ promoter.
4. Vector as claimed in claim 2, in which the translation initiation region is λcIIrbs.
5. Vector as claimed in claim 2, in which the region consists of all or part of the sequence :

ATAACACAGGAACAGATCTATG.

6. Vector as claimed in one of claims 1 to 5, in which the promoter used is the P_L promoter.
7. Vector as claimed in one of claims 1 to 6, which contains, in addition, a transcription antitermination function.
8. Vector as claimed in claim 7, in which the transcription antitermination function is the λN gene.
9. Vector as claimed in one of claims 1 to 8, which contains the origin of replication of pBR322.
10. Vector as claimed in one of claims 1 to 9, which contains a gene coding for resistance to an antibiotic.
11. Vector as claimed in claim 10, which contains a gene for resistance to ampicillin.
12. Bacteria transformed by a vector as claimed in one of claims 1 to 11.
13. Bacteria as claimed in claim 12, which are E. coli.
14. Process for preparing human γ interferon, wherein a bacterium as claimed in one of claims 12 and 13 in cultured on a culture medium, and wherein the human γ-LFN obtained after culturing is isolated.

**Patentansprüche**

1. Expressionsvektor des fertigen Proteins von γ-Interferon in Bakterien von dem Typ, der die Nukleotidsequenz, die für das fertige Protein von Human-γ-Interferon codiert, und die Plasmidelemente, die die Expression dieser Nukleotidsequenz sicherstellen, enthält, dadurch gekennzeichnet, daß das 5´-Ende der Sequenz, die für das fertige Protein codiert, die folgende ist :

5´ATG TGC TAC TGT CAG GAT CCC 3´
TAC ACG ATG ACA GTC CTA GGG
Met Cys Tyr Cys Gln Asp Pro

2. Klonierungsvektor nach Anspruch 1, dadurch gekennzeichnet, daß er außerdem :
   a) den Replikationsursprung eines bakteriellen Plasmids,
   b) einen Promotor,
   c) einen Initiationsbereich der Übersetzung, der den Codon ATG der Sequenz, die für fertiges IFN-γ codiert, enthält,
enthält.
3. Vektor nach Anspruch 2, dadurch gekennzeichnet, daß er :
   a) den Replikationsursprung eines bakteriellen Plasmids,
   b) einen Promotor der Bakteriophage λ, P_L, P_R oder P´_R
   c) einen Initiationsbereich der Übersetzung, ausgewählt unter λcIIrbs, vollständig oder teilweise, oder einer synthetischen Sequenz, die unter die Kontrolle des Promotors der Bakteriophage λ gebracht ist,
enthält.
4. Vektor nach Anspruch 2, dadurch gekennzeichnet, daß der Initiationsbereich der Übersetzung λcIIrbs ist.
5. Vektor nach Anspruch 2, dadurch gekennzeichnet, daß der Bereich aus der ganzen oder teilweisen Sequenz :

ATAACACAGGAACAGATCTATG

besteht.

6. Vektor nach einem der Ansprüche 1 bis 5, dadurch gekennzeichnet, daß der verwendete Promotor der Promotor $P_L$ ist.

7. Vektor nach einem der Ansprüche 1 bis 6, dadurch gekennzeichnet, daß er außerdem eine Antiterminationsfunktion der Transkription enthält.

8. Vektor nach Anspruch 7, dadurch gekennzeichnet, daß die Antiterminationsfunktion der Transkription das Gen $\lambda N$ ist.

9. Vektor nach einem der Ansprüche 1 bis 8, dadurch gekennzeichnet, daß er den Replikationsursprung von pBR322 enthält.

10. Vektor nach einem der Ansprüche 1 bis 9, dadurch gekennzeichnet, daß er ein Gen enthält, das für die Resistenz gegenüber einem Antibiotikum codiert.

11. Vektor nach Anspruch 10, dadurch gekennzeichnet, daß er ein Resistenzgen gegen Ampicillin enthält.

12. Bakterien, die durch einen Vektor nach einem der Ansprüche 1 bis 11 transformiert sind.

13. Bakterien nach Anspruch 12, dadurch gekennzeichnet, daß es sich um E. coli handelt.

14. Verfahren zur Herstellung von Human-γ-Interferon, dadurch gekennzeichnet, daß man eine Bakterie nach einem der Ansprüche 12 und 13 auf einem Nährboden kultiviert hat und daß man nach der Kultivierung das erhaltene Human-IFN-γ isoliert.

FIG.1

FIG_2

FIG_3

FIG_4

CTGAAGATCAGCTATTAGAAGAGAAAGATCAGTTAAGTCCTTTGGACCTGATCAGCTTGATACAAGAACTACTGATTTCAACTTCTTTGGCTTAATTCTCTCGGAAACG

EcoRⅡ
ATG AAA TAT ACA AGT TAT ATC TTG GCT TTT CAG CTC TGC ATC GTT TTG GGT TCT CTT GGC TGT TAC TGC CAG GAC CCA TAT GTA AAA GAA
Met Lys Tyr Thr Ser Tyr Ile Leu Ala Phe Gln Leu Cys Ile Val Leu Gly Ser Leu Gly Cys Tyr Cys Gln Asp Pro Tyr Val Lys Glu

GCA GAA AAC CTT AAG AAA TAT TTT AAT GCA GGT CAT TCA GAT GTA GCG GAT AAT GGA ACT CTT TTC TTA GGC ATT TTG AAG AAT TGG AAA
Ala Glu Asn Leu Lys Lys Tyr Phe Asn Ala Gly His Ser Asp Val Ala Asp Asn Gly Thr Leu Phe Leu Gly Ile Leu Lys Asn Trp Lys

GAG GAG AGT GAC AGA AAA ATA ATG CAG AGC CAA ATT GTC TCC TTT TAC TTC AAA CTT TTT AAA AAC TTT AAA GAT GAC CAG AGC ATC CAA
Glu Glu Ser Asp Arg Lys Ile Met Gln Ser Gln Ile Val Ser Phe Tyr Phe Lys Leu Phe Lys Asn Phe Lys Asp Asp Gln Ser Ile Gln

AAG AGT GTG GAG ACC ATC AAG GAA GAC ATG AAT GTC AAG TTT TTC GAT AGC AAC AAA AAG AAA CGA GAT GAC TTC GAA AAG CTG ACT AAT
Lys Ser Val Glu Thr Ile Lys Glu Asp Met Asn Val Lys Phe Phe Asp Ser Asn Lys Lys Lys Arg Asp Asp Phe Glu Lys Leu Thr Asn

TAT TCG GTA ACT GAC TTG AAT GTC CAA CGC AAA GCA ATA CAT GAA CTC ATC CAA GTG ATG GCT GAA CTG TCG CCA GCA GCT AAA ACA GGG
Tyr Ser Val Thr Asp Leu Asn Val Gln Arg Lys Ala Ile His Glu Leu Ile Gln Val Met Ala Glu Leu Ser Pro Ala Ala Lys Thr Gly

AAG CGA AAA AGG AGT CAG ATG CTG TTT CAA GGT CGA AGA GCA TCC CAG TAA TGG TTG TCC TGC CTG CAA TAT TTG AAT TTT AAA TCT AAA
Lys Arg Lys Arg Ser Gln Met Leu Phe Gln Gly Arg Arg Ala Ser Gln ***

TCTATTTATTAATATTTAACATTATTTATATGGGGAATATATTTTTAGACTCATCAATCAAATAAGTATTTATAATAGCAACTTTTGTGTAATGAAAATGAATATCTATTAATATATGTA

TTATTTATAATTCCTATATCCTGTGACTGTCTCACTTAATCCTTTGTTTTCTGACTAATTAGGCAAGGCTATGTGATTACAAGGCTTTATCTCAGGGGCCAACTAGGCAGCCAACCTAAG

Sau3A
CAAGATCCCATGGGTTGTGTGTTTATTTCACTTGATGATACAATGAACACTTATAAGTGAAGTGATACTATCCAGTTACTGCCGGTTTGAAAATATGCCTGCAATCTGAGCCAGTGCTTT

AATGGCATGTCAGACAGAACTTGAATGTGTCAGGTGACCCTGATGAAAACATAGCATCTCAGGAGATTTCATGCCTGGTGCTTCCAAATATTGTTGACAACTGTGACTGTACCCAAATGG

AAAGTAACTCATTTGTTAAAATTATCAATATCTAATATATATGAATAAAGTGTAAGTTCACAACTAAAAAAAAAAAAAAAA

## FIG.5

CONSTRUCTION DE PTG909

FIG_6

CONSTRUCTION DE PTG941

IFN

PTG909

*MetCysTyrCysGlnAspProTyr*
TAAGGAAGTACTTA<u>CATATG</u>TGTTATTGCCAGGACC<u>CATATG</u>....
            N ᴅᴇI                     N ᴅᴇI

N ᴅᴇ I

+

TATGTGCTACTGTCAGGATCCC
ACACGATGACAGTCCTAGGGAT

IFN

PTG941

*MetCysTyrCysGlnAspProTyr*
TAAGGAAGTACTTA<u>CATATG</u>TGCTACTGTCA<u>GGATCC</u>CTAT
              Nᴅᴇ I              BᴀᴍHI

# FIG_7

7

CONSTRUCTION DE PTG951

FIG_8